# EUROPÄISCHE PATENTSCHRIFT

(11) **EP 2 703 038 B1**
(45) Veröffentlichungstag und Bekanntmachung des Hinweises auf die Patenterteilung: **20.04.2016**
(21) Anmeldenummer: 13003612.2
(22) Anmeldetag: 18.07.2013
(51) Int. Cl.: A61N 1/05

(54) **Elektrodenanordnung mit einer drei hohle Kanäle aufweisenden Zuleitung und mit einer Positionierungs-Fixierungsvorrichtung**
Electrode assembly with a feed line with three hollow channels and with a positioning and fixation device
Ensemble d'électrodes avec une conduite d'amenée présentant trois canaux creux et avec un dispositif de positionnement et d'attachement

(30) Priorität: 27.08.2012 DE 102012016764
(43) Veröffentlichungstag der Anmeldung: 05.03.2014
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- EP-A2- 0 965 359
- DE-A1- 3 523 226
- DE-A1- 19 930 268
- DE-U1-202010 015 348
- US-A1- 2006 178 030

## Beschreibung

Die Erfindung betrifft eine Elektrodenanordnung, die die exakte Positionierung und Fixierung von implantierbaren epikardial platzierten Elektroden nach einer Herzoperation ermöglicht und die eine Defibrillation (kardioversion) und Stimulation und gegebenenfalls zusätzlich eine Messung oder Überwachung des Herzens, insbesondere des linken Vorhofs in Verbindung mit einem außerhalb des Körpers anschließbares Herzschrittmacher oder Defibritlator ermöglicht und wobei die Elektroden nach erfolgter Anwendung jederzeit leicht durch Zug entfernbar sind.

Vorhofflimmern tritt mit zunehmendem Lebensalter und nach Herzoperationen relativ häufig auf und ist einer der wichtigsten Gründe für die postoperative Morbidität. Insgesamt scheint Vorhofflimmern im Allgemeinen und besonders nach Herzoperationen in den letzten Jahren zugenommen zu haben, was mit einem immer älter werdenden Patientenkollektiv erklärt wird. Liberaturangaben über postoperatives Vorhoffimmern bei Patienten mit bisherigem Sinushythmus ergaben ein durchschnittliches Auftreten in 30% - 40% nach Bypass-Operationen am Herzen.

Vorhofflimmern führt zu einer schnellen Überleitung der Erregung auf die Ventrikel, so dass es zu akuter hämodynamischer Instabilität kommen kann. Die bisherige angewandte elektrische Kardioversion ist eine nicht-medikamentöse und sehr effektive Methode zur Wiederherstellung des Sinusrhythmus, die allerdings eine Kurznarkose zur Voraussetzung hat. Diese Kurznarkose kann jedoch gerade bei Patienten nach Bypass-Operation die bestehenden neuronalen Probleme (Vigilanz) durch die soeben überstandene Herzoperation verschärfen, was zu einer verlängerten Aufwachphase führen kann oder sogar eine erneute intubation mit maschineller Beatmung erforderlich macht Ein weiteres Problem stellt die Antikoagulation bei postoperativen Patienten mit Vorhofflimmern dar. Wenn die Rhythmusstörung länger als 24 Stunden anhält, ist eine Antikoagulation erforderlich, um die Thrombenbildung mit der Gefahr eines Schlaganfalls zu reduzieren. All diese Faktoren führen zu einem komplizierten postoperativen Verlauf bei Patienten nach Bypass-Operation, der sich in einem um ca. 5 Tage verlängerten Krankenhaus Aufenthalt mit vermehrten Kosten niederschlägt Vorhofflimmern kann während der Liegedauer des Patienten auf der Intensivstation wiederholt auftreten. Zur Prophylaxe wird in der Literatur das Multisite Pacing beschrieben. Hierbei wird besonders der linke Vorhof an mehreren Arealen gleichzeitig simuliert. Für das Multisite Pacing werden z.B. unipolare Herzdrähte verwendet, die gegenüber einer äußeren Anode betrieben werden.

### Problem

Die Positionierung und Fixierung von Kardioversions-und/oder Stimutationsetektroden am linken Vorhof bereitet wegen der speziellen anatomischen Lage des linken Vorhofs erhebliche Schwierigkeiten. Der linke Vorhof befindet sich auf der Rückseite des Herzens und ist somit für die Positionierung und für eine temporäre Fixierung der Elektroden schwer zugänglich. Daher wird auf die Kardioversion und Stimulation des linken Vorhofs oft verzichtet trotz seiner großen medizinischen Bedeutung für die Prophylaxe und Therapie des Vorhofflimmerns.

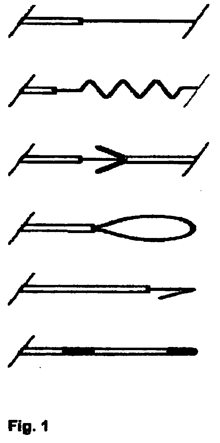

Wie wird das Problem bisher gelöst:
Für die temporäre Fixierung der Elektroden im Herzmuskel sind in Fig.1 die verschiedenen heute gebräuchlichen Methoden dargestellt. Das Verhaken der Elektroden über diverse Anker, Loops oder Zick-Zack-Anordnungen ist aber aufgrund der Tatsache, dass man den linken Vorhof bei einer offenen Herzoperation nicht sehen kann, sehr erschwert. Eine falsche oder ungenaue Positionierung der Elektroden bringt nicht den gewünschten Erfolg. Das Dokument EP 0 965 359 A2 offenbart eine Elektroden anordnung mit das Metallwenden ausgebildeten Kardioversionselektroden und mit einem implantierbaren Katheren mit Kanälen für die zuleitungsdramse der Elektroden.

Es besteht daher die Aufgabe, eine Elektrodenanordnung bereitzustellen, die die Implantation von epikardial plazierten Elektroden nach einer Herzoperation erlaubt, wobei sich die Elektroden sowohl zur Kardioversion als auch zur Stimulation separat an jedem Vorhof eignen und sich vor ellem leicht am linken Vorhof positionieren und fixieren lassen.

Diese Aufgabe wird mit den Mitteln und Merkmalen des Schutzanspruches 1 gelöst.

Die erfindungsgemasse Elektrodenanordnung ermöglicht die exakte Positionierung und Fixierung der Elektroden sowie eine weitgehend schmerzfreie Defibrillation (Kardioversion), ein Stimulation und gegebenenfalls zusätzlich eine Messung oder Überwachung des Herzens. insbesondere des linken Vorhofs in Verbindung mit einem außerhalb des Körpers anschließbaren Herzschrittmacher oder Defibrillator. Die Elektroden sind nach erfolgter Anwendung jederzeit leicht durch Zug entfernbar.

Die Elektrodenanordnung umfasst mindestens zwei großflächig angeordneten Katdioversionselektrode (1,2) und eine weitere Stimulationselektrode mit mindestens einem Pol (3), wobei die Kardioversionselektroden Metallwendeln sind, die in Form eines Tennisschlägen angeordnet sind. Die Stimulationselektrode ist zwischen den Kardioversionselektroden (1.2) angeordnet Zwischen den voneinander isolierten Polen der Elektroden (1,2) kann die Kardioversion erfolgen und zwischen einer der Elektroden (1 oder 2) und einem Pol der Elektrode (3) kann die Stimulation des Herzens erfolgen.

Die Elektrodenanordung besitzt eine Zuleitung (4) mit mindestens drei hohlen Kanälen durch die am proximalen Ende (11) die Zuleitungsdrähte (10,18) für die Kardioversionselektroden sowie eine Positionierungs-Fixterungsvorrichtung bestehend aus einem Führungskanal (5) und einem Stilett (6) eingeführt werden. Das Stilett wird in den Führungskanal (5) eingeschoben und ist geführt verschiebbar bis zum distalen Ende (7) der Zuleitung (4).

Der Führungskanal (5) ist Metallwender aus mindestens einem Draht ausgebildet und bildet gleichzeitig die Zuleitung für die Stimulationselektrode (3).

Das Stilett ist ein biegsamer Draht aus Metall oder Kunststoff, vorzugsweise aus Edelstahl oder Nitinol, vorzugsweise mit einem Durchmesser von 0,25-0,5mm.

Die Zuleitung (4) ist ein isolierender hochflexibler Schlauch.

Durch Führungskanal (5) mit Stilett (6) ist die Zuleitung (4) versteift, sodass die Elektrodenanordnung gegenüber bekannten Anordnungen, die alle hoch flexibel sind, die notwendige Stabliltät erhält um gezlelt auf der Rückseite des Herzens bis zum linken Vorhof geschoben zu werden. Ist die richtige Position erreicht, wird durch gezielte Biegung des Stiletts die temporäre epikardiale Fixierung der Elektroden auf dem linken und/oder rechten Vorhof erreicht. Der Ausdruck "Position ierungs-Fixierungsvorrichtung" beschreibt genau diesen Vorgang. Nach erfolgter Fixierung wird das Stilett entfernt und die Zuleitung (4) im Brust- oder Bauchraum mit Hilfe eines Ligaturverschlusses (9) fixiert.

Die erfindungsgernässe Elektrodenanordnung erlaubt die Positionierung und Federung jeder epikardial platzierten Elektrode sowohl am rechten als auch am linken Vorhof. Durch die gezielte Positionierung und Biegung ist eine Anpassung an die Anatomie der Vorhöfe gegeben.

Die Zuleitung (4) wird über eine Öffnung im Brust- oder Bauchraum nach außen geführt und durch einen Ligaturveschluss fixiert. Als Ligaturveschluss eignen sich alle im Handel erhältlichen Ligaturverschlüsse.

Ebenso geeignet ist der in DE20 2011 109 277 beschriebene Ligaturveschluss. Hierbei wird die Körperöffnung, aus der die Elektrodenzuleitung herausragt, mit einem Kunststoffseil fixiert Das Kunststoffteil enthält eine zentrale Öffnung und wird auf die Körperöffnung geklebt, sodass eine feste und dichte Verbindung erfolgt Die Elektrodenzuleitung (gegebenenfalls mehrere Elektrodentuleitungen) wird durch die Ausgangstülle einer abnehmbaren Kappe geführt und mit Hilfe eines Ligaturveschlusses fixiert.

Fig. 6 zeigt den aus DE20 2011 109 277U1 Fig. 3 bekannten Ligaturveschluss. Zusätzlich zur Fixierung der Elektrodenzuleitung verhindert der Ligaturveschluss, die Elektrodenzuleitung bei Bewegungen des Patienten unbeabsichtigt wieder in das Innere des menschlichen Körpers gelangt und dort eventuell eine Infektion hervorruft.

Der Ausdruck großflächige Kardioversionselektrode bedeutel, dass die beiden Elektroden (1,2) einen ausreichenden Abstand haben müssen um überhaupt eine Kardioversion durchführen zu können. Der Abstand sollte beispielsweise mehr als 1 cm betragen. Die Kardioversionsetektroden sind in Form eines Tennisschlägers angeordnete Metallwendeln.

Damit die bei der elektrischen Kardioversion/Stimulation auftretende Feldstärkeverteilung ausschließlich in die Richtung des Herzens wirksam wird, wird der dem Herzen abgewandte Teil der Elektroden (abgewandte Teil des Tennisschlägers) vorteiihefterweise durch eine dünne Kunststoffmembran (7) isoliert. Als Isolationsmaterial eignet sich besonders eine Folie aus Silikon.

Dadurch dass die Kardioversion mit Elektroden, die temporär an den beiden Vorhöfen epikardial und lokal fixiert sind, vorgenommen wird, reduziert sich die notwendige elektrische Energieabgabe zur Cardioversion erheblich.

Durch Einsatz von Elektroden den Elektrodenanordung entsprechen Schutzanspruch 1 ergibt sich die Möglichkeit einer Unterdrückung von erneutem Auftretendes Vorhofflimmerns (Multisite Pacing) ohne Narkose und für den Patienten weitgehend schmerzfrei. Später lassen sich diese Elektroden - wie bei den üblich heute benutzten Herzdrähten - leicht entfernen.

### Abbildungen:

Weitere Einzelheiten, Merkmale und Vorteile der Erfindung werden anhand der schematischen Zeichnungen näher erläutert
**Fig.1** zeigt verschiedene distate Enden der heute gebräuchlichen temporären Elektroden für die Stimulation des Herzens nach einer Herzoperation in Form von Metalllitzen mit unterschiedlichen Fixierungen.
**Fig.2** zeigt die erfindungsgemässe Elektrodenanordnung positioniert und fixiert am linken Vorhof.
**Fig.3** zeigt die Elektrodenanordnung positioniert und fixiert am linken Vorhof. Das Stilett ist entfernt. Die Öffnung des Brustraums ist durch eine Ligatur verschlossen.
**Fig.** 4 zeigt die in Form eines Tennisschlägers angeordneten Metallwendeln, die der Kardioversion dienen, die Stimulationselektrode und die Zuleitung.
**Fig.5** zeigt die Positionierungs-Fixierungsvorrichtung.
**Fig. 6** zeigt die in DE20 2011 109 277 U1 beschriebenen Ligaturverschlüsse.

### Beschreibung der Abbildungen:

**Fig.1** zeigt heute gebräuchliche temporäre Elektroden für die Stimulation des Herzens nach einer Herzoperation in Form von Metalllitzen mit unterschiedlichen Fixierung (T-bar, Loop, Anker, Zick-zack),

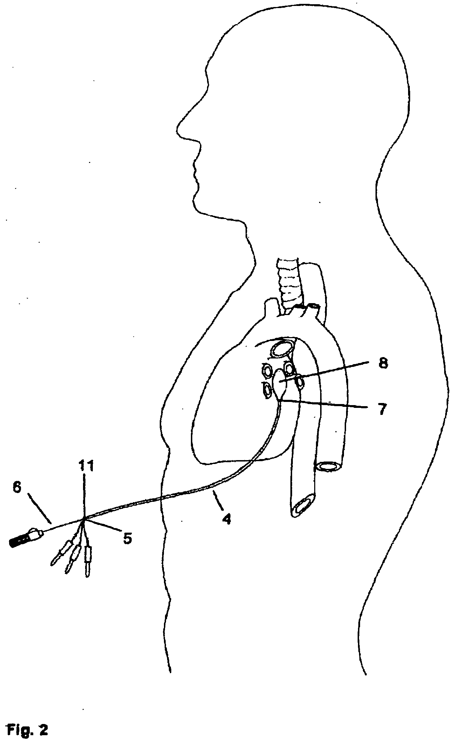

**Fig.2** zeigt die erfindungsgemasse Elektrodenvorrichtung. Die als Metallwendeln ausgebildeten Kardioversionselektroden sind in Form eines Tennisschlägers angeordnet und am linken Vorhof positioniert. Die Elektroden befinden sich auf der dem Herzen zugewandten Seite und sind auf dieser Übersichtsdar stellung nicht sichtbar Damit die bei der elektrischen Kardioversion/Stimulation auftretende Feldstärkeverteilung ausschließlich in die Richtung des Herzens wirksam wird, ist der dem Herzen abgewandte Teil des Tennisschlägers durch eine dünne Kunststoffmembran (8) isoliert Die Kunststoffmembran ist beispielsweise eine Silikonfolie oder eine Polyurethanfolie.
   Die Elektrodenanordung verfügt Ober eine Zuleitung (4), die am proximale Ende (11) drei Zuleitungsdrähte für die beiden Kardioversionselektroden und für die Stimulationselektrode hat. Das Stilett (6) wird über das proximale Ende (11) in den Führungskanal (5) eingeführt und versteift die Zuleitung und ist geführt verschiebbar bis zum distalen Ende (7) der Zuleitung (4).
   Die Zuleitung (4) ist ein isolierender hochflexibler und somit biegsamer Kunststoffschlauch, beispielsweise ein Silikonschlauch oder Polyurethanschlauch.

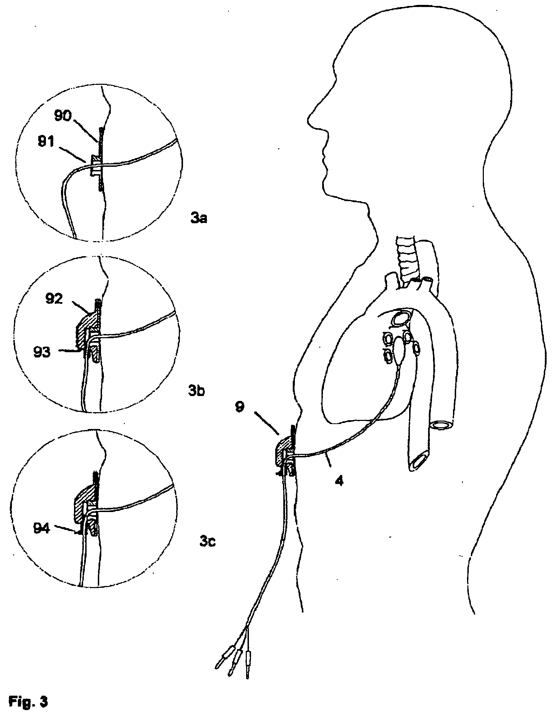

**Fig. 3** die epikardial platzierten Elektroden positioniert und fixiert am linken Vorhof. Das Stilett (6) ist entfernt. Die Öffnung des Brustraums ist durch eine Ligatur (9) verschlossen. Die Ligatur bewirkt eine zusätzliche Fixierung.
**Fig. 3a-3c** zeigt einen geeigneten Ligaturverschluss im Detail vergleichbar mit dem in DE20 2091 109 277 U1 Fig. 3 beschriebenen Verschtuss.
**Fig. 3a** zeigt ein Kunststoffteil (90) vorzugsweise aus Silikon mit einer zentralen Öffnung (91). Der Kunststoffteil ist radial geschlitzt und wird außen auf die Körperöffnung nach dem Durchzug der Zuleitung (4) aufgeklebt Danach wird die Zuleitung (4) durch die Kappe (92) und durch die Ausgangstülle (93) geführt **(****Fig. 3b****).** Die Kappe (92) wird mit dem Kunststoffteil (90) verbunden, beispielsweise durch einen Schnappverschluss verbunden.
**Fig. 3c** zeigt wie die Zuleitung (4) mit Hilfe der Ligatur (94) an der Ausgangstülle fixiert wird. Ein Verschieben der Elektrode ist auch bei Bewegung des Patienten jetzt nicht mehr möglich.

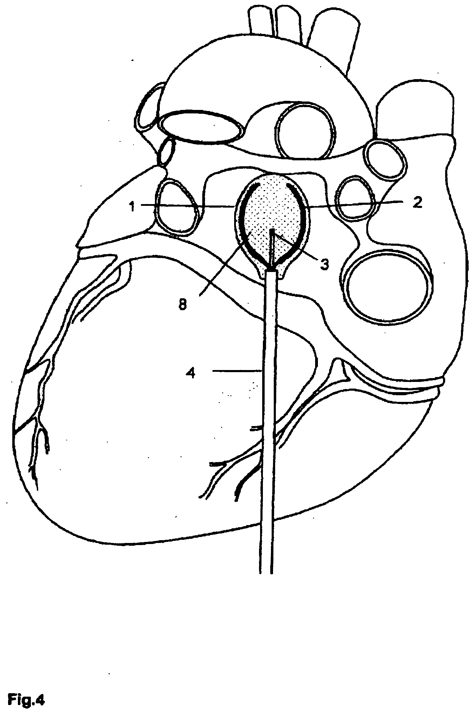

**Fig. 4** zeigt die in Form eines Tennisschlägers großflächig angeordneten Metaltwendeln (1,2) die der Kardioversion dienen, die Stimulationselektrode mit dem Pol (3) und die Zuleitung (4). Auf der dem Herzen abgewandten Seite befindet sich die Isolationsmembran (8). Zwischen den beiden voneinander isolierten Polen der Wendel (1,2) erfolgt die Kardioversion. Die Stimulation des Herzens kann zwischen dem Pol (3) und der Wendel (1) oder der Wendel (2) erfolgen.

In einer weiteren Ausführungsform ist die Stimulationselektrode bipolar oder multipolar, sodass die Stimulation zwischen den einzelnen Polen untereinander erfolgen kann. Auch die Kardioversionselektroden können multipolar sein und beispielsweise aus vier Metallwendeln bestehen.,

Wichtig ist, dass bei der erfindungsgemäßen Anordnung aufgrund der großflächig angeordneten Elektroden (Metallwendeln) in Form eines Tennisschlägen die Defibrillation unmittelbar und separat an jedem Vorhof erfolgen kann. Die für die Defibrillation benötigten Sockenergien liegen unter 5 Joule, vorzugsweise unter 2 Joule, besonders bevorzugt unter 1 Joule, sodass die Defibrillation weitgehend schmerzfrei erfolgen kann.

Ebenso erlaubt die erfindungsgemäße Anordnung eine Stimulation ohne dass eine weitere Stimulationselektrode fixiert werden muss.

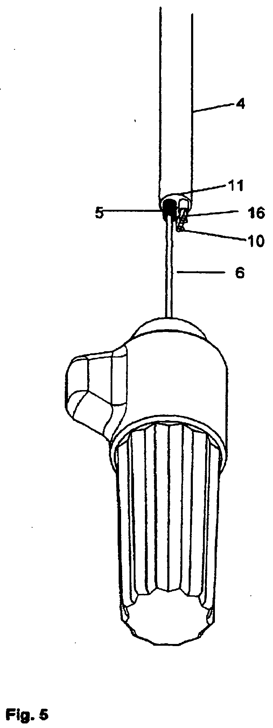

**Fig. 5** ist die zentrale Figur. Sie zeigt Zuleitung (4) im Detail. Im Inneren der Zuleitung (4) verlaufen drei hohle Kanäle durch welche am proximalen Ende (11) die elektrischen Zuleitungen (10,16) für die beiden voneinander isolierten Kardioversionselektroden sowie eine Positionierungs-Fixierungsvorrichtung bestehend aus einem als Metallwendel ausgebildeten Führungskanal (5) und einem Stilett (6) eingeführt werden.

Der Führungekanal (5) ist als Metallwendel ausgebildet und bildet, falls eine Stimulation erforderlich ist, die Zuleitung zur Stimulationselektrode. Das Stilett ist ein biegsamer Draht aus Metall oder Kunststoff, vorzugsweise Nitinokiraht.

Das Stilett kann ebenso wie die Metallwendel (5) vorgeformt sein gebogen order werden. Die Verformung erfolgt entweder vor der Positionierung und/oder bei Positionierung der Elektroden. Durch die Verformung erfolgt die erste Fixierung der Elektroden am Vorhof. Es besteht die Möglichkeit einer feinen Nachjustierung der Positionierung und Fixierung, die unproblematisch durchfahrbar ist. Dadurch ist ein optimaler Sitz der Elektroden am Vorhof möglich und jede anatomische Lage der Vorhöfe lässt sich meistern.

Unterschiedliche Ausgestaltung der erfindungsgemässen Elektrodenanordnung: Materialauswahl: Edelstahl, Platin, Gold, Elgiloy, Nitinol, Isotan, elektrisch leitfähige Faden, Karbonfäden, elektrisch leitfähiger Kunststoff, auch mit Nanopartikeln gemischt.

Isolation: Polyurethan, Polyethylen, Silikon, PTFE, Pebax, Polyamid, Peek, alle biokompatiblen Kunststoffe incl. Lacke, z.B. Polyimid.

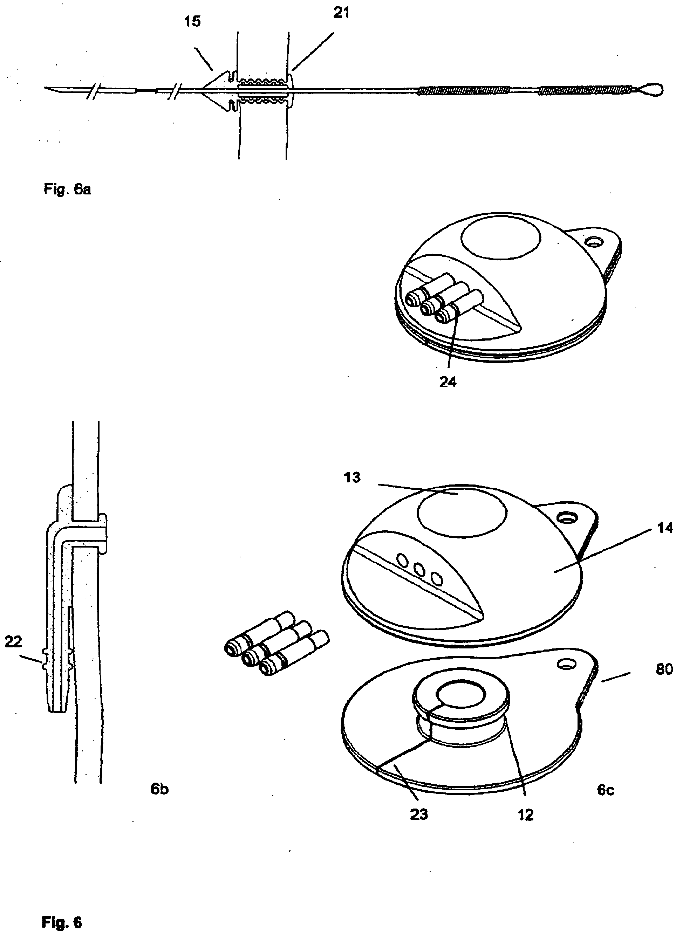

**Fig. 6** zeigt die in DE 20 2011 109 277U1 beschriebenen Ligaturverschlüsse.
**Fig. 6a** zeigt einen dehnbaren Ligaturverschluss 15 mit Innenscheibe 21 aus weichem Kunststoff.
**Fig. 6b** zeigt die Ligatur (22) an der Ausgangstülle.
**Fig. 6c** entspricht Fig. 3 aus DE20 2011 109 277. Die untere Platte (80) ist radial geschlitzt (23) und besteht aus einem weichen Kunststoffmaterial, vorzugshalber Silikon und wird außen auf die Körperöffnung nach dem Durchzug der Zuleitung aufgeklebt. Danach wird der weitere Teil der Zuleitung durch die Kappe (14) und durch einen der Ausgänge geführt. Nachdem die Kappe mit dem unteren Teil (80) durch denn Schnappverschluss (12) verbunden ist, kann der übrige Teil der Zuleitung mit einer Ligatur an den Ausgangstüllen (24) fest verbunden werden. Ein transparentes Fenster (13) in der Kappe (14) ermöglicht die Kontrolle und Entnahme von eventuell austretender Köperflüsslgkeit.

## Patentansprüche

1. Elektrodenanordnung bestehend aus
mindestens zwei Kardioversionselektroden (1,2) mit Zuleltungsdrähten (10, 16), wobei die Kardioversionselektroden (1,2) Metallwendeln sind;
und
einer uni- bi -oder multipolaren Stimulationselektrode mit mindestens einem Pol (3), die zwischen den Elektroden (1,2) angeordnet ist, wobei zwischen den voneinander isolierten Polen der Elektroden (1,2) die Kardioversion erfolgen kann und wobei zwischen einer der Elektroden (1 oder 2) und einem Pol der Elektrode (3) die Stimulation des Herzens erfolgen kann; und
einer Positionierungs-Fixierungsvorrichtung: und
eine Zuleitung (4), die mit einem Ligaturverschluss (9) verbindbar ist und durch den Ligaturverschluss an einer Öffnung im Brustraum order Bauchraum fixierbar ist ; wobei die Elektrodenanordnung eine Zuleitung (4) besitzt mit mindestens drei hohlen Kanälen, wobei durch zwei dieser Kanäle am proximalen Ende (11) die Zuleitungsdrähte (10.16) für die Kardioversionselektroden elnführbar sind und durch den dritten Kanal die Positionlerungs-Fixierungsvorrichtung einführbar ist, **dadurch gekennzeichnet, dass** die Metallwendeln in Form eines Tennisschlägen angeordnet sind und dass die Positionienungs-Fixierungsvorrichtung aus einem Führungskanal (5) und einem Stilett (6) in Form eines biegsamen Drahtes besteht, wobei der Führungskanal (5) als Metallwendel ausgebildet ist und gleichzeitig die Zuleitung für die Stimulationselektrode (3) bildet.

2. Elektrodenanordnung gemäß Anspruch 1, wobei das Stilett (6) ein biegsamer Draht aus Metall oder Kunststoff, vorzugsweise aus Edelstahl oder Nitinol ist.

3. Elektrodenanordnung gemäß Anspruch 1, wobei die Zuleitung (4) ein Kunststoffschlauch, vorzugsweise ein Silikonschlauch oder Polyurethanschlauch ist.

4. Elektrodenanordnung gemäß Anspruch 1, wobei die Elektrode (3) multipolar ist und die Stimulation des Herzens zwischen den einzelnen Polen der multipolaren Elektrode erfolgen kann.

5. Elektrodenanordnung gemäß Anspruch 1, wobei der vom Herzen abzuwendende Teil der Kardioversions-/Stimulationselektroden durch eine dehnbare Isolierende Folie, vorzugsweise Silikonfolie oder Polyurethanfolle, abgedeckt ist.

6. Elektrodenanordnung gemäß einem der Ansprüche 1-5, wobei die Elektroden aus Edelstahl, Platin, Gold, Elgiloy, Nitinol, Isotan, elektrisch leitfählgen Fäden, Karbonfäden, elektrisch leitfähigem Kunststoff, auch mit Nanopartikeln gemischt, bestehen.

## Claims

1. Electrode arrangement comprising
at least two cardioversion electrodes (1,2) with lead wires (10, 16), wherein the cardioversion electrodes (1,2) are metallic coils; and
an uni-, bi- or multipolar stimulation electrode with at least one pole (3), said pole being located between the electrodes (1,2), whereby cardioversion can be performed between the poles (1,2) said poles being isolated from each other; and whereby stimulation of the heart can be performed between one of the electrode poles (1 or 2) and electrode pole (3); and
a positioning and fixation device;
and a supply line (4), which is connectable with a ligature closure (9), said supply line being fixable through the ligature closure at an opening in the thoracic cavity or abdomen; wherein the electrode arrangement has a supply line (4) with at least three hollow channels, wherein the lead wires (10, 16) of the cardioversion electrodes are insertable through two of these channels at the proximal end (11) and the positioning and fixation device is insertable through the third channel, **characterized in that** the metallic coils are arranged in form of a tennis racket and that the positioning and fixation device consists of a guide channel (5) and a stylet (6) in the form of a flexible wire, wherein the guide channel (5) is formed as a metallic coil and simultaneously constitutes the supply line for the stimulation electrode (3).

2. Electrode arrangement according to claim 1, wherein the stylet (6) is a flexible wire made of metal or plastic, preferably made of stainless steel or Nitinol.

3. Electrode arrangement according to claim 1, wherein the supply line (4) is a plastic tube, preferably a silicone or polyurethane tube.

4. Electrode arrangement according to claim 1, wherein the electrode (3) is multipolar and the stimulation of the heart can be performed between each pole of the multipolar electrode.

5. Electrode arrangement according to claim 1, wherein the part of the cardioversion/stimulation electrode facing away from the heart is covered by a flexible insulating film, preferably a silicone or polyurethane film.

6. Electrode arrangement according to one of the claims 1-5, wherein the electrodes are made of stainless steel, platinum, gold, Elgiloy, nitinol, isotan, electrically conductive filaments, carbon threads, electrically conductive plastic, also mixed with nanoparticles.

## Revendications

1. Ensemble d'électrode comportant
au moins deux électrodes de cardioversion (1,2) avec des fils conducteurs (10, 16), dans lequel les électrodes de cardioversion (1,2) sont des spirales métalliques ; et
une électrode de stimulation uni-, bi- ou multipolaire avec au moins un pôle (3) étant disposé entre les électrodes (1, 2) ; la cardioversion peut être effectuée entre les pôles des électrodes (1, 2) isolés les uns des autres et la stimulation du coeur peut être effectuée entre l'une des électrodes (1 ou 2) et un pôle de l'électrode (3); et
un dispositif de fixation et de positionnement ; et
une ligne d'alimentation (4) qui peut être connectée à une fermeture ligaturée (9) et qui grâce la fermeture ligaturée peut être fixée à une ouverture dans la cage thoracique ou dans l'abdomen; et
l'ensemble d'électrode possède une ligne d'alimentation (4) avec au moins trois canaux creux, dans lequel les fils conducteurs des électrodes de cardioversion (10, 16) sont insérables à travers deux de ces canaux à l'extrémité proximale (11) et le dispositif de fixation et de positionnement est insérable à travers le troisième canal, **caractérisé en ce que** les spirales métalliques sont disposées sous la forme de raquette de tennis et que le dispositif de fixation et de positionnement se compose d'un canal de guidage (5) et d'un stylet (6) sous la forme d'un fil flexible, dans lequel le canal de guidage (5) est formé d'une spirale métallique et constitue simultanément la ligne d'alimentation pour l'électrode de stimulation (3).

2. Ensemble d'électrode selon la revendication 1, dans lequel le stylet (6) est un fil flexible en métal ou plastique, de préférence en acier inoxydable ou en Nitinol.

3. Ensemble d'électrode selon la revendication 1, dans lequel la ligne d'alimentation (4) est un tuyau souple en matière plastique, de préférence un tuyau en silicone ou en polyuréthane.

4. Ensemble d'électrode selon la revendication 1, dans lequel l'électrode (3) est multipolaire et la stimulation du coeur peut avoir lieu entre chacun des pôles de l'électrode multipolaire.

5. Ensemble d'électrode selon la revendication 1, dans lequel la partie des électrodes de cardioversion et de stimulation à éviter du coeur est couverte d'un film extensible isolant, de préférence un film en silicone ou polyuréthane.

6. Ensemble d'électrode selon une des revendications 1-5, dans lequel les électrodes sont composées d'acier inoxydable, platine, or, elgiloy, Nitinol, isoton, fils électriquement conducteurs, fibres de charbons, plastique électriquement conducteur, aussi mélangé à des nanoparticules.
